# EUROPEAN PATENT SPECIFICATION

(11) **EP 3 649 934 B1**
(45) Date of publication and mention of the grant of the patent: **30.12.2020**
(21) Application number: 18460066.6
(22) Date of filing: 21.11.2018
(51) Int. Cl.: A61B 5/053, G06T 11/00

(54) **A METHOD OF IMAGE RECONSTRUCTION IN ELECTRICAL IMPEDANCE TOMOGRAPHY**
VERFAHREN ZUR BILDREKONSTRUKTION IN DER ELEKTRISCHEN IMPEDANZ-TOMOGRAFIE
PROCÉDÉ DE RECONSTRUCTION D'IMAGES POUR LA TOMOGRAPHIE PAR IMPÉDANCE ÉLECTRIQUE

(30) Priority: 08.11.2018 PL 42769718
(43) Date of publication of application: 13.05.2020
(73) Proprietor: Uniwersytet w Bialymstoku, 15-328 Bialystok (PL)
(72) Inventor: Szymanski, Krzysztof R., 15-337 Bialystok (PL); Walczyk, Cezary J., 15-635 Klepacze (PL)

(56) References cited:
- SUN ZHENG-HANG ET AL: "Two-dimensional electrostatic model for the Van der Pauw method", PHYSICS LETTERS A, vol. 381, no. 27, 9 May 2017 (2017-05-09), pages 2144-2148, XP085040948, ISSN: 0375-9601, DOI: 10.1016/J.PHYSLETA.2017.04.020

## Description

The present invention relates to a method of image reconstruction in electrical impedance tomography, based on a theory of flows in multiply connected domains.

Electrical impedance tomography (EIT) is a method of imaging a spatial distribution of a specific resistivity of an interior of a sample. In this method, electrical current is induced between two electrodes and potential difference is measured on two other electrodes. The measurements are performed for various electrodes positioned according to a predetermined arrangement at an edge of an investigated area of the sample, to obtain a set of currents and potentials to be used for image reconstruction.

EIT is applied i.a. in medicine (for imaging of interior of a body, in particular for monitoring lungs inflammation), in geology (for investigating of resistivity anomalies of mineral deposits) in ecology (for imaging of water deposits or pollution leakage from waste deposits to ground). EIT may be also applied for monitoring filling of vessels and pipelines, for imaging defects of structures (cracks, losses) or for imaging quality of concrete.

An image of impedance distribution can be constructed as a solution of a so-called inverse problem.

The solution of the inverse problem in EIT was presented in "An Introduction to the Mathematical Theory of Inverse Problems" (Andreas Kirsch, Springer Science+Business Media, LLC 2011, page 167). The inverse problem was solved using integral operators acting on the infinite dimension data space and was defined on a simply-connected domain, i.e. a domain containing no holes. There is no theory related to multiply connected domains. Multi-connected domains are related to systems/samples/materials important for applications, particularly those containing cracks, holes and losses in homogeneous materials.

Practically, only finite sets of data are available in EIT. There are a few methods of image reconstruction based on advanced numerical procedures.

One of the known methods of solving the inverse problem is regularization, that involves approximation of an infinite spectrum of operators by constructing a finite series of operators acting in a finite dimension space. The method is described in the publication "A regularization technique for the factorization method" (Armin Lechleiter, Inverse Problems 22 (2006), pages 1605-1625).

There is known a method suitable for applications in dynamical conditions using back-projection concepts. An image is repeatedly corrected by differences obtained from subsequent iterations. Such method is presented in the US patent US4617939 and in the publication "Applied potential tomography" (D. C. Barber and B. H. Brown, J. Br. Interplanet. Soc., 1989, vol. 42, no. 7, str. 391-393).

There is known a static method of construction of images of a spatial distribution of resistivity using nonlinear optimization methods. The method is presented in a US patent US5465730 and in the publication "Comparing Reconstruction Algorithms for Electrical Impedance Tomography" (T. J. Yorkey, J. G. Webster, and W. J. Tompkins, IEEE Trans. Biomed. Eng., 1987, vol. BME-34, no. 11, pages 843-852).

The algorithms dedicated for image reconstruction require explicit specification of a shape and an anisotropy of the sample. To incorporate this information into an algorithm, calculations are performed using a finite element method. By the finite element method, approximate solutions of partial differential equations are achieved. The investigated domain is divided into smaller sections, in which solutions are approximated by a set of known functions. The calculations are performed only at the selected nodes of the sections. By the finite element method, currents and potentials are calculated for selected distributions of resistivity and these data are used for image construction, as described in the publication "A finite element model with node renumbering for adaptive impedance imaging" (E. J. Woo, P. Hua, W. J. Tompkins, and J. G. Webster, Proc. IEEE EMBC, 1988, vol.1, pages 277-278).

Image reconstruction procedures in EIT involve a difficult problem of inversion of nearly singular matrices. One of the procedures for treatment of these cases is called singular value decomposition and is presented the European patent EP0599877B1 and in the publications "An interleaved drive electrical impedance tomography image reconstruction algorithm" (B. M. Eyiiboglu, Physiol. Meas. 17 (A), 1996, pages 59-71) and "Time series of EIT chest images using singular value decomposition and Fourier transform" (N Kerrouche, CN McLeod, W.R.B. Lionheart, Physiol Meas. 2001 Feb; 22(1), pages147-57).

There is known a method of image reconstruction based on the Bayessian principles. The measured results are treated as random variables and a solution of the inverse problem is formulated based on a probability theory, as presented in the publication "Statistical inversion and Monte Carlo sampling methods in EIT" (J. Kaipio, V. Kolehmainen, E. Somersalo, and M. Vauhkonen, Inverse problems 16, 2000, str. 1487-1522).

Recently, the problem of currents and potential distributions in a doubly connected domain (i.e. a domain with a single hole) was solved and published in the paper "Determination of the Riemann modulus and sheet resistance of a sample with a hole by the van der Pauw method" (K. Szymański, K. 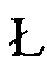 apiński, J. L. Cieśliński, Measurement Science and Technology, 26 (2015) 055003). Also, a triply connected domain (i.e. a domain with two holes) was analysed and results were published in "Determination of topological properties of thin samples by the van der Pauw method" (K. R. Szymański, C. J Walczyk, J. L. Cieslinski, submitted to "Measurement" international journal, 28.04.2018, Ref: MEAS-D-18-01040). These publications relate to determination of some geometrical properties by measurement of extreme resistances using four contacts moving on an edge of the sample.

An article "Two-dimensional electrostatic model for the Van der Pauw method" (by Sun Zheng-Hang et al, Physics Letters A, col. 381, no. 27, pages 2144-2148, ISSN: 0374-9601) describes use of a two-dimensional electrostatic model to explore Van der Pauw (VdP) method on samples with holes.

There is a need to provide alternative methods of image reconstruction in EIT. These alternative methods are desirable in particular if based on theoretical assumptions that are different than commonly used, because results of EIT examinations sometimes are not unique. Therefore, results of different methods allow to establish more reliable diagnostics.

The object of the invention is a method of image reconstruction in EIT (Electrical Impedance Tomography) to determine inhomogeneities of a sample, the method comprising: positioning contacts at an edge of an investigated domain of the sample; inducing flow of current through the contacts; and reading potentials on the contacts, by using a system of connections connected between the contacts and a data acquisition system. The method is characterized by: determining a conformal map that represents a transformation of a shape of a disk to a shape of the investigated domain; providing a plurality of hole positions within the investigated domain; processing the currents and potentials read at the contacts using a theory of flows in multiply connected domains by means of a processor connected to the data acquisition system and determining radiuses of holes at the provided positions; imaging the determined radiuses of holes by means of a visualisation system, by transforming the positions and radiuses of holes to the investigated area using the conformal map.

The method may comprise determining radiuses of holes for at least two sets of randomly provided hole positions.

The method may comprise determining radiuses of holes for hole positions provided along a regular array.

The method according to the present invention is presented by means of example embodiments on a drawing, in which:
Fig. 1 shows contacts located at points at an edge of the multiply connected domain D;
Fig. 2A-2C show examples of contact positions for samples with different shapes.
Thin lines show connections for current and voltage signal transmission.
Fig. 3 show an embodiment of the method according to the invention.
Fig. 4A shows an example of a homogeneous sample with two holes and Fig. 4B shows a reconstructed image of that sample.
Fig. 5A shows an example of a sample with two halves that differ in specific resistivity and Fig. 5B shows a reconstructed image of that sample.

For a better understanding of the present invention, principles of the theory of flows in the multiply connected domains are presented below.

Any multiply connected domain can be conformal mapped into a domain with some number of concentric holes. The statement follows from the Riemann theorem and is presented for example in a monograph "Complex Analysis" (L. V. Ahlfors, 1979, 3rd ed. New York: McGraw-Hill). By "multiply connected domain" we understand concentric or not concentric openings, as well as slits in a solid object.

Because currents and potentials are invariant under the conformal map, without loss of generality, let's consider *k* holes that are circular and not intersecting and have radiuses *r*₁, *r*₂*,* ... *r*ₖ ∈*R*¹, centred at points *o*₁, *o*₂,.. *o*ₖ ∈ *R*², and located in a unit circle. Our system, abbreviated by *D,* is thus a domain with *k*+1 edges, *k* edges resulting from the holes and *k*+1th edge of the unit circle.

We present how to find electrical potential *V*(*x,y*) within the domain *D* resulting from the current source and the current sink located at points *s*₊*, s*₋ ∈ *D.* We use a method of image charges, known in electrostatics and described in the publication "The Feynman Lectures on Physics" (R. Feynman, R. Leighton, M. Sands, vol. 2, Addison-Wesley-Longman, 1970)) and construct a set of points *S*(*s*₊) and *S*(*s₋*) at which image sources and sinks are located. For every already mentioned edge *i, i*=1,...*k*+1*,* we define a function *Iᵢ*: *R*²*→R*²*,* which is an inversion: ${I}_{i} \left(x\right) = \frac{x - {o}_{i}}{\left(x-{o}_{i}\right) ⋅ \left(x-{o}_{i}\right)} {r}_{i}^{2} + {o}_{i} .$ (1)

For *i*=*k*+1 obviously *o*_{*k*+1}=0 and *r*_{*k*+1}=1*.* Next, we construct an image of a set *X* in a map being a sum of all inversions *Iᵢ*, *s: X→ Y* $\left(X\right) = \left\{{y}_{i}:{y}_{i}={I}_{i}\left({x}_{j}\right),i=1,2,…k+1,{x}_{j}∈X\right\} .$ (2)

Starting from any point *p∈D,* by an iterated procedure we define infinite series of sets *s*⁰, *s*¹, *s*²....: ${s}^{0} = \left\{p\right\} , {s}^{1} = s \left({s}^{0}\right) , {s}^{2} = s \left({s}^{1}\right) , … {s}^{n + 1} = s \left({s}^{n}\right) .$ (3)

Finally, an image *S*(*p*) of a single point *p* is an infinite set of points: $S \left(p\right) = {∪}_{i = 0}^{∞} {s}^{i} .$ (4)

Electrical potential at point *q*=(*x,y*) of the infinite plane with a point source located at *s*₊ and a point sink located at *s*₋ is equal to: $V \left(q, {s}_{+}, {s}_{-}\right) = \frac{jρ}{2 π h} ln \frac{\left|{s}_{+}-q\right|}{\left|{s}_{-}-q\right|} ,$ (5) wherein *j* is a current of the source, *p* is a specific resistivity and *h* is a thickness of the plate with *k* considered holes. Electrical potential at any point *q* ∈ *D,* for the domain *D* with the source located at *s*₊ and the sink located at point *s*₋, equals to a contribution of potentials resulted from the sources and the sinks located at the points belonging to *S*(*s*₊) i *S*(*s₋*): $V \left(q\right) = {\sum }_{\begin{matrix}{t}_{+} ∈ S \left({s}_{+}\right) \\ {t}_{-} ∈ S \left({s}_{-}\right)\end{matrix}} V \left(q, {t}_{+}, {t}_{-}\right) = \frac{jρ}{2 π h} ln {\prod }_{\begin{matrix}{t}_{+} ∈ S \left({s}_{+}\right) \\ {t}_{-} ∈ S \left({s}_{-}\right)\end{matrix}} \frac{\left|{t}_{+}-q\right|}{\left|{t}_{-}-q\right|} .$ (6)

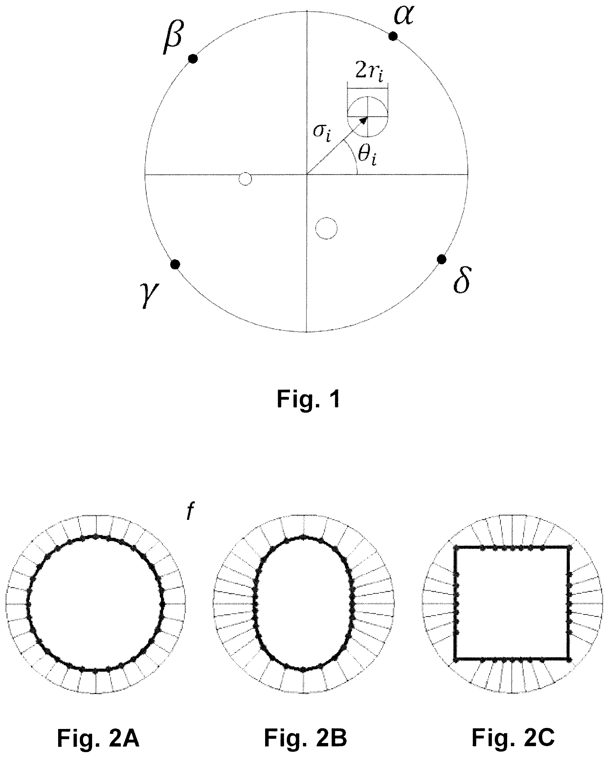

The explicit form of formula (6) is not known, sets *S*(*s*₊) and *S*(*s₋*) are usually fractals. Approximation of (6) for small holes is interesting for the invention. Let us consider four point contacts located on the edge of the domain at angles *α β γ δ* (shown in Fig. 1)

The current *j_{αβ}* enters the sample at the contact *α* (source) and leaves at the contact *β* (sink), whereas the potential difference *V_{γδ}* is measured between the contacts *γ* and *δ*. The four probe resistance for the contacts *α β γ δ* is defined as *R_{αβγδ}*=*V_{γδ}*/*j_{αβ}.* By expanding the right hand side of (6) into a power series in small parameters *r*₁, *r*₂, ... *r*ₖ and inserting currents, the four probe resistance is approximated by ${R}_{αβγδ} = {R}_{αβγδ}^{0} + {\sum }_{i = 1}^{n} {r}_{i}^{2} f \left(α, β, γ, δ, {σ}_{i}, {θ}_{i}\right) + O \left({r}_{i}^{2}{r}_{j}^{2}\right) ,$ (7) wherein ${R}_{αβγδ}^{0}$ is the four probe resistance for a disk without holes: ${R}_{αβγδ}^{0} = λ ln \left|\frac{sin \frac{\left(γ-α\right)}{2} sin \frac{\left(δ-β\right)}{2}}{sin \frac{\left(γ-β\right)}{2} sin \frac{\left(δ-α\right)}{2}}\right| ,$ (8) and the parameter *λ*=*ρ*/(*πh*)*.*

The expansion coefficients in formula (7) are functions of contact positions and positions of canters of holes. The explicit form is a rational expression of trigonometric functions: $f \left(α, β, γ, δ, {σ}_{i}, {θ}_{i}\right) = λ ⋅ \frac{{a}_{1}}{{a}_{2}} \left({a}_{3}-{a}_{4}\right) ,$ (9) wherein abbreviations *a*₁*, a*₂*, a*₃*, a*₄ are introduced for clarity: ${a}_{1} = 16 ln 2 ⋅ sin \frac{α - β}{2} sin \frac{γ - δ}{2} ,$ $\begin{matrix}{a}_{2} = \left(1-2σcos\left(α-θ\right)+{σ}^{2}\right) \left(1-2σcos\left(β-θ\right)+{σ}^{2}\right) \\ ⋅ \left(1-2σcos\left(γ-θ\right)+{σ}^{2}\right) \left(1-2σcos\left(δ-θ\right)+{σ}^{2}\right) ,\end{matrix}$ $\begin{matrix}{a}_{3} = 2 σ \left(1+{σ}^{2}\right) \\ ⋅ \left(cos\frac{β - δ}{2}cos\frac{α + γ - 2 θ}{2}+cos\frac{α - γ}{2}cos\frac{β + δ - 2 θ}{2}\right)\end{matrix}$ $\begin{matrix}{a}_{4} = 2 {σ}^{2} cos \frac{α + β + γ + δ - 4 θ}{2} + \left(1+{σ}^{4}\right) cos \frac{α + β - γ - δ}{2} \\ + 4 {σ}^{2} cos \frac{α - β}{2} cos \frac{γ - δ}{2} .\end{matrix}$

In the expansion (7) of the exact expression (6), the coefficients of components of type *rᵢrⱼ* and *rᵢrⱼrₖ* (*i≠j≠k≠i*) are equal to 0. As a consequence, expansion (7) is valid up to the fourth powers of radiuses and contains only squares of radiuses, which is important for the concept of image construction. Expansion (7) is an explicit expression for currents and potentials in multiply connected domain, i.e. a homogeneous material with small holes. For arbitrary location of holes and contacts positions, expansion (7) depends on the squares of radiuses only, thus is linear in squares of these parameters. By comparing the right hand side of (7) with an experimental data for four probe resistances, one may optimise the radiuses to obtain the best fit to the data. Using a known method of least squares, a set of equations for optimization has a form of: $\begin{matrix}{x}^{T} Ax + b = 0 , \\ {x}_{i} \geq 0 ,\end{matrix}$ (10) wherein *A* is a positively defined, symmetric matrix. Coordinates *xᵢ* are squares of the holes radiuses *rᵢ*, and this is the reason for the non-negativity of *xᵢ* in formula (10). The solution of formula (10) is known in mathematical optimization as a nonlinear programming problem with Karush, Kuhn and Tucker conditions, with a solution presented in the publication "Nonlinear programming" (H. W. Kuhn, A. W. Tucker, 1951, Proceedings of 2nd Berkeley Symposium, Berkeley: University of California Press., pages 481-492). The solution of formula (10) allows one to approximate continuous distribution of impedance by finite distribution of holes.

The method according to the invention originates from the known theory of conformal mapping and can be used for image reconstruction of objects of any shape. This is particularly important for technical applications. From the theory of complex numbers there are known conformal maps transforming a disk into for example an oval, a square or a rectangle. In the method according to the invention it is easy to define positions of contacts for an arbitrary shape (for example, an oval or a square or a rectangle) and to transform, by this conformal map, parameters of the holes obtained from expansion (7). Therefore, in the method according to the invention it is not necessary to perform simulations by finite element methods to adapt or to change the algorithm when one changes the shape of the investigated sample. In the method according to the invention, the algorithm for image reconstruction is independent on the investigated shape, only the appropriate conformal map has to be used in the visualisation.

Figs. 2A-2C show examples of contact positions, marked by dots, for different shapes of investigated samples.

Fig. 2A shows equally spaced contacts located on the edge of the disk.

Using a conformal map *f*: $f \left(z\right) = i ⋅ \frac{z}{1 - {\left(az\right)}^{2}} , 0 < a < 1 ,$ (11) one get positions of contacts for EIT of a sample with an oval shape, shown in Fig. 2B.

Using a conformal map *f*: $f \left(z\right) = \frac{1}{a} ⋅ F \left(arc sin z,-1\right) , a = F \left(arc sinh\frac{1 + i}{\sqrt{2}},-1\right) ,$ (12) wherein *F* is an elliptic integral of the first kind known from mathematical analysis, one gets positions of contacts for EIT of a sample with a square shape, shown in Fig. 2C.

In all cases of the different shapes shown in Figs. 2A-2C, the algorithm for image reconstruction is exactly the same, the only difference are positions of contacts and visualisation by the use of the conformal mapping.

For consistency of presentation of the method of the invention, it shall be mentioned that in the case of investigation of samples with a shape of a disk (Fig. 2A), the conformal map for visualisation has the form of the an identity function, i.e. *f*(*z*)=*z*.

The method according to the invention can be used for imaging of inhomogeneities of impedance by EIT. Details of inhomogeneous sample are approximated by a uniform sample with distribution of small holes mimicking inhomogeneity and parameters of the holes are used for image construction.

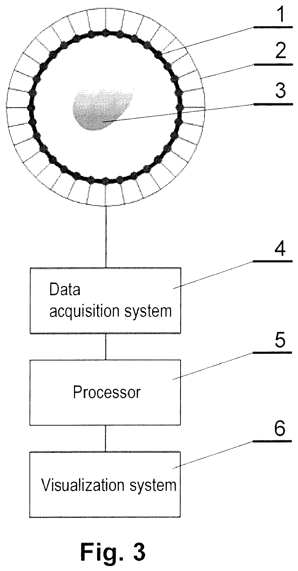

An example embodiment of the method according to the invention is presented in Fig. 3. Contacts (1) with induced currents and resulting voltages are located on the edge of the sample and are connected by a system of cables (2) to data acquisition system (4). Inhomogeneity (3) inside the investigated sample is shown schematically. Induction of currents and reading of voltages (AC or DC) is performed as known in EIT techniques. Measured data are processed using the method according to the invention by a processor (5) and sent to the visualisation system (6), wherein an appropriate conformal map is used for image visualisation.

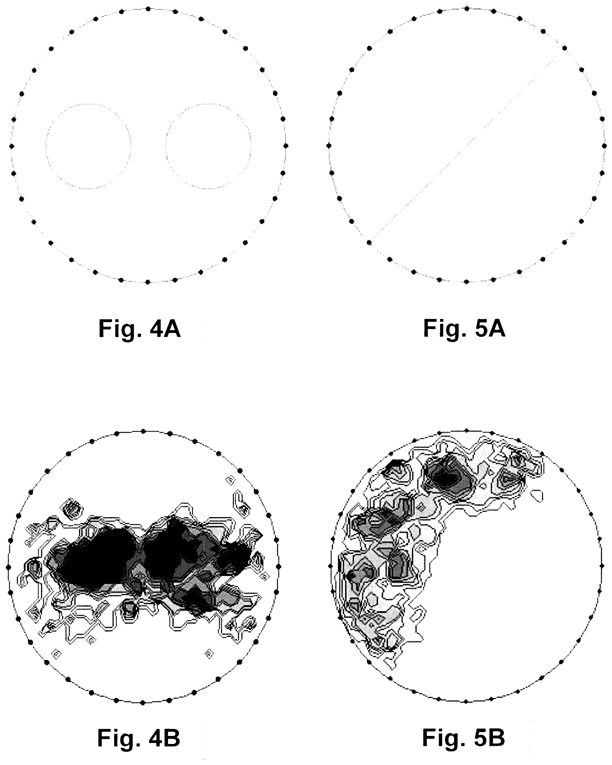

Using the finite element method, currents and potentials for provided distributions were calculated. Then, using the method according to the invention, radiuses of holes with randomly generated positions were fitted to obtain the best consistency with the calculated data for samples shown schematically in Figs. 4A and 5A.

In one embodiment, a plurality of sets, each comprising a plurality of randomly generated positions of holes, can be provided. For each set, radiuses of holes can be calculated using the theory of flows in multiconnected domain and the process can be repeated for each set. Finally, the results may be averaged.

In another embodiment, a set of hole positions arranged along a uniform mesh can be provided, for example a regular array of holes within the investigated domain of the sample.

As a result of operation of the algorithm presented herein, for positions wherein the data read by the data acquisition system 4 indicate presence of a hole, relatively large values of radiuses are output, while for positions, wherein the data read by the data acquisition system 4 indicate absence of a hole, relatively small or even zero values of radiuses are output.

Examples of image reconstruction by generation of distribution of holes and visualisation, are shown in Fig. 4A-4B and 5A-5B. Fig. 4A shows an example of a homogeneous disk with two holes, while Fig. 4B shows a reconstructed image that is visualised using the method according to the invention (in this case, two large holes were reconstructed as a dense distribution of many small holes located in the region of large holes). Fig. 5A shows an example of a disk with two halves that differ in their specific resistivity by a factor of two. Fig. 5B shows a reconstructed image (in this case small holes are located on the half with a larger specific resistance).

One advantage of the method according to the invention is its mathematical simplicity that results in a fast algorithm. An image is constructed using the theory without the need of operators acting in a space with infinite dimensions. Therefore, one may expect easy implementation of the method into fast processors.

A favourable feature of the method according to the invention is the possibility of operating the algorithm repeatedly with different sets of the hole positions, to use obtained distributions of radiuses to increase precision.

Another favourable feature of the method according to the invention is its theoretical assumption that is quite different from those revealed up to date. It was already demonstrated, that there are possibilities of cloaking some details making them invisible in EIT imaging, as described in the publication "Impedance Imaging, Inverse Problems, and Harry Potter's Cloak" (K. Bryan and T. Leise, Society for Industrial and Applied Mathematics Vol. 52, No. 2, pp. 359-377). It is thus evident, that EIT methods of imaging could not guarantee unique results. The method according to the invention, based on nonconventional assumptions of the theory of flows in multiconnected domains, may be used to limit ambiguities in EIT imaging.

## Claims

1. A method of image reconstruction in EIT (Electrical Impedance Tomography) to determine inhomogeneities of a sample, the method comprising:
- positioning contacts (1) at an edge of an investigated domain of the sample;
- inducing flow of current through the contacts (1); and
- reading potentials on the contacts (1), by using a system of connections (2) connected between the contacts (1) and a data acquisition system (4);
the method **characterized by**:
- determining a conformal map (*f*) that represents a transformation of a shape of a disk to a shape of the investigated domain;
- wherein the contacts (1) are positioned at the edge of the investigated domain at positions which are calculated from equally spaced contacts located on an edge of the disk using the conformal map (*f*);
- providing a plurality of hole positions within the disk;
- by means of a processor (5) connected to the data acquisition system (4), processing the currents and potentials read at the contacts (1) by:
- selecting four (*α*, *β ,γ, δ*) of the contacts and measuring a four-probe resistance between the selected contacts (*R_{αβγδ}*=*V_{γδ}*/*j_{αβ}*);
- determining what radiuses of holes at the provided hole positions provide a best fit for the measured four-probe resistance; and
- imaging the determined radiuses of holes that are the best fit by means of a visualisation system (6), by transforming the positions and radiuses of these holes to the investigated domain using the conformal map (*f*).

2. The method of claim 1, comprising determining radiuses of holes for at least two sets of randomly provided hole positions.

3. The method of claim 1 comprising determining radiuses of holes for hole positions provided along a regular array.

## Patentansprüche

1. Im Verfahren der Bildrekonstruktion in der Elektrischen Impedanz-Tomografie zur Bestimmung von Inhomogenität des Materials:
- werden am Rand des zu untersuchenden Bereichs Kontakte positioniert (1),
- durch die der Stromfluss gezwungen wird;
- und an den Kontakten Spannungen durch das System der Leitungen gemessen (2), die die Kontakte mit einem Daten-Erfassungs-System (4) verbinden.
Das Verfahren zeichnet sich dadurch aus, dass:
- eine konforme Abbildung (*f*) bestimmt wird, die eine Transformation von einer Scheibe in die Gestalt des zu untersuchenden Bereichs abbildet;
- die Kontakte am Rand des zu untersuchenden Bereichs an Positionen angebracht werden, die durch die Wirkung der Transformation (*f*) auf gleich entfernte Positionen bestimmt sind,
- viele Positionen der Löcher des zu untersuchenden Bereichs geliefert werden,
- an den Kontakten (1) der Stromfluss und die Spannungen unter Anwendung eines Prozessors (5) verbunden mit dem Daten-Erfassungssystem (4) gemessen werden, so dass
- vier Kontakte an den Positionen (*α, β, γ, δ*) ausgewählt werden und der Vier-Punkt-Widerstand (*R_{αβγδ}*=*V_{γδ}*/*j_{αβ}*)*,* bestimmt wird,
- die Radien der Löcher an den gelieferten Positionen anhand der besten Anpassung der Radien der Löcher der erhaltenen Vier-Punkt-Widerstände bestimmt werden
- die bestimmten Radien der Löcher mit Hilfe eines Visualisierung-Systems (6) abgebildet werden, durch die Transformation der Positionen und Radien der Löcher in dem zu untersuchenden Bereich unter Anwendung der konformen Abbildung (*f*).

2. Das Verfahren entsprechend des Patentanspruchs 1, zeichnet sich dadurch aus, dass die Radien von Löchern für mindestens zwei Sätze beliebig gelieferter Positionen bestimmt werden.

3. Das Verfahren entsprechend des Patentanspruchs 1, zeichnet sich dadurch aus, dass die Radien von Löchern für gelieferte Positionen verteilt im regulären Netz bestimmt werden.

## Revendications

1. Le procédé de reconstruction d'images en tomographie d'impédance électrique pour
déterminer l'hétérogénéité du matériel, dans lequel procédé :
- des contacts sont répartis sur le bord de la zone du matériel à examiner (1) ;
- à travers lesquels le flux de courant est forcé ;
- et les tensions par le biais du système de fils (2) connectés entre les contacts et le système d'acquisition de données (4) sont lues ;
**caractérisé en ce que**
- la représentation conformationnelle *(f)* représentant la transformation du disque à la forme de la zone examinée est définie ;
- les contacts sur la côte de la zone examinée sont placés à des positions déterminées par l'action de la conversion (*f*) en positions équidistantes
- de nombreuses positions de trous dans la zone examinée sont définies ;
- à l'aide du processeur (5) et du système d'acquisition de données (4), les courants et les tensions aux contacts (1) sont lus de sorte que
- quatre contacts sont sélectionnés dans des positions (α, β, γ, δ) et la résistance à quatre points (*R*αβγδ=*V*γδ/jαβ) est déterminée,
- les rayons des trous dans les positions prédéfinies sont déterminés sur la base
du meilleur ajustement des rayons des trous des résistances
à quatre points obtenues
- et les rayons des trous désignés sont représentés au moyen d'un système de visualisation (6), en transformant les positions et les rayons des trous dans la zone examinée à l'aide de la représentation conformationnelle *(f)*.

2. Le procédé selon la revendication 1 **caractérisé en ce que** les rayons des trous sont déterminés pour au moins deux ensembles de positions définies de manière aléatoire.

3. Le procédé selon la revendication 1 **caractérisé en ce que** les rayons des trous
pour les positions définies distribuées dans un réseau régulier sont déterminés.
